(19) Europäisches Patentamt
European Patent Office
Office européen des brevets

(11) EP 3 252 048 A1

(12) EUROPEAN PATENT APPLICATION
published in accordance with Art. 153(4) EPC

(43) Date of publication:
**06.12.2017 Bulletin 2017/49**

(21) Application number: **16742708.7**

(22) Date of filing: **25.01.2016**

(51) Int Cl.:
***C07D 403/06*** *(2006.01)* ***A61K 31/404*** *(2006.01)*
***A61P 35/00*** *(2006.01)* ***A61P 1/16*** *(2006.01)*

(86) International application number:
**PCT/CN2016/071951**

(87) International publication number:
**WO 2016/119646 (04.08.2016 Gazette 2016/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.01.2015 CN 201510045099**

(71) Applicant: **Sound Biopharmaceuticals Ltd.**
**Guangzhou 510310 (CN)**

(72) Inventors:
- **WANG, Zhong**
  **Guangzhou**
  **Guangdong 510235 (CN)**
- **LI, Qing**
  **Guangzhou**
  **Guangdong 510235 (CN)**

(74) Representative: **J A Kemp**
**14 South Square**
**Gray's Inn**
**London WC1R 5JJ (GB)**

# (54) SUNITINIB PRODRUG AND PHARMACEUTICAL COMPOSITION

(57) The present invention provides a Sunitinib prodrug having formula I, wherein $R_{12}$, $R_{13}$ are selected from the group consisting of H, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl and 5-15 membered heteroaryl; $R_{14}$ is selected from the group consisting of R', OR', SR' and $N(R')_2$; and R' is selected from the group consisting of H, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl, 5-15 membered heteroaryl, hydroxyl $C_1$-$C_6$ alkyl, carboxyl $C_1$-$C_6$ alkyl, $C_1$-$C_6$ alkyl amido and phosphate group. The Sunitinib prodrug of the invention provides better pharmacokinetics and pharmacodynamics, better safety and lower toxicity.

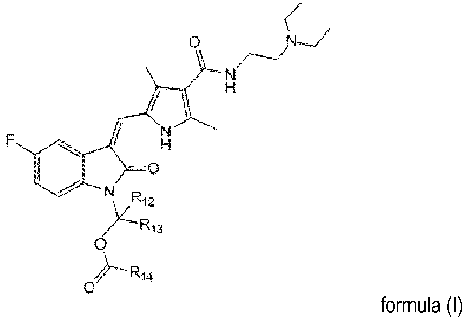

formula (I)

EP 3 252 048 A1

## Description

### CROSS-REFERENCE TO RELATED APPLICATION

This application claims the priority of Chinese Patent Application Number 201510045099.1, with the title of "Sunitinib Prodrug", filed on 28 January 2015 in the name of Ascenta Pharmaceuticals Inc., the entirety of which is incorporated herein by reference.

### FIELD

The present disclosure relates to prodrug compounds of Sunitinib, a pharmaceutical composition comprising any of the prodrug compounds, and relates to use of these compounds.

### BACKGROUND

Sunitinib is a multi-target kinase inhibitor which has been approved by the FDA to treat metastatic renal cell carcinoma (MRCC) and gastrointestinal stromal tumor (GIST). Currently available product on the market is Sunitinib malate, whose structure is:

Sunitinib is a multi-kinase inhibitor which prevents vascular endothelial growth factor receptors (VEGFRs), platelet derived growth factor receptor, stem cell factor receptor (c-Kit), FMS-like tyrosine kinase-3 and glial cell line derived neurotrophic factor receptor (RET). The multi-target kinase inhibitor can target against cancer cells and tumor microenvironment simultaneously, inhibiting multiple individual or crossed signal pathways, and therefore can inhibit complicated polymorphic lesions that drive tumor growth and survival. It is more likely to provide a significant effect on cancers having complicated mechanism, especially solid tumors, compared to single targeting agents.

However, Sunitinib fails to show effect on many other cancers, including liver cancer, in clinical trials. The main reason is treatment-related toxicity. For example, Sunitinib has significant toxicity for 50% of patients with kidney cancer.

### SUMMARY

In some embodiments, provided herein is a Sunitinib prodrug having formula I

formula (I)

wherein:

$R_{12}$, $R_{13}$ are selected from the group consisting of H, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl and 5-15 membered heteroaryl;

$R_{14}$ is selected from the group consisting of R', OR', SR' and $N(R')_2$; and

R' is selected from the group consisting of H, $C_1$-$C_6$alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl, 5-15 membered heteroaryl, hydroxyl $C_1$-$C_6$ alkyl, carboxyl $C_1$-$C_6$ alkyl, $C_1$-$C_6$ alkyl amido and phosphate group.

In some embodiments, $R_{12}$, $R_{13}$ are H, and $R_{14}$ is selected from the group consisting of N,N-dimethylaminomethyl, t-butyl, phenyl, p-fluorophenyl, biphenyl, dimethylamino, cyclopropyl, methyl, ethyl, propyl, isopropyl, butyl, isobutyl, cyclobutyl, pentyl, isopentyl, neopentyl, cyclopentyl, 1-methylcyclobutyl, N-methylamino, N-ethylamino, N,N-methylethylamino, n-hexyl, cyclohexyl, methylthio, ethylthio, propylthio, isopropylthio, cyclopropylthio, butylthio, isobutylthio, cyclobutylthio, methoxy, ethoxy, propoxy, isopropoxy, butoxy, cyclobutoxy, pentylamino, pentylthio, pentyloxy, isopentylamino, neopentylamino, t-butylamino, cyclopentylamino, cyclopentylthio, cyclopentyloxy, cyclohexylamino, cyclohexylthio, cyclohexyloxy, p-methoxyphenyl, p-chlorophenyl and o-fluorophenyl.

Compounds can be formulated as or be in the form of pharmaceutically acceptable salts. Contemplated pharmaceutically acceptable salt forms include, without limitation, mono, bis, tris, tetrakis, and so on. Pharmaceutically acceptable salts are non-toxic in the amounts and concentrations at which they are administered. The preparation of such salts can facilitate the pharmacological use by altering the physical characteristics of a compound without preventing it from exerting its physiological effect. Useful alterations in physical properties include lowering the melting point to facilitate transmucosal administration and increasing the solubility to facilitate administering higher concentrations of the drug.

In some embodiments, provided herein is a pharmaceutically acceptable salt of a compound of formula I. Acids that can be formed into a salt with the compound of formula I include, without limitation, an inorganic acid, such as hydrochloric acid, sulfuric acid; an organic acid, such as acetic acid, trifluoroacetic acid, citric acid, maleic acid, oxalic acid, succinic acid, benzoic acid, tartaric acid, fumaric acid, mandelic acid, ascorbic acid or malic acid; and an amino acids, such as alanine, aspartic acid, lysine; a sulfonic acid, such as methanesulfonic acid, p-toluenesulfonic acid.

In some embodiments, provided herein is a pharmaceutical composition comprising a compound of formula I or a salt thereof, wherein the pharmaceutical composition further comprises a pharmaceutically acceptable carrier.

In some embodiments, provided herein is a use of a compound of formula I or a salt thereof in preparation of a drug for treating a cancer. In one embodiment, the cancer is metastatic renal cell carcinoma, gastrointestinal stromal tumor or liver cancer. In one embodiment, the compound is N-[2-(diethylamino)ethyl]-5-[(5-fluoro-2-oxo-1-(N,N-dimethylamino)methylcarboxymethyl-2,3-indolin-3-ylmeth ylene)]-2,4-dimethyl-1 H-pyrrolidine-3-carboxamide (Compound **A**); N-[2-(diethylamino)ethyl]-5-[(5-fluoro-2-oxo-1-(t-butylcarboxymethyl)-2,3-indolin-3-ylmethylene)]-2,4-dimethyl -1H-pyrrolidine-3-carboxamide (Compound **1**); or N-[2-(diethylamino)ethyl]-5-[(5-fluoro-2-oxo-1-(phenylcarboxymethyl)-2,3-indolin-3-ylmethylene)]-2,4-dimethyl -1H-pyrrolidine-3-carboxamide (Compound **2**); ***N***-[2-(diethylamino)ethyl]-5-[(5-fluoro-2-oxo-1-(1-methylcyclobutylcarboxymethyl)-2,3-indolin-3-ylmethylene)]-2,4-dimethyl-1***H***-pyrrolidine-3-carboxamide (Compound **3**); ***N***-[2-(diethylamino)ethyl]-5-[(5-fluoro-2-oxo-1-(N-methylaminocarboxymethyl)-2,3-indolin-3-ylmethylene)]-2,4-dimethyl-1***H***-pyrrolidine-3-carboxamide (Compound **4**); ***N***-[2-(diethylamino)ethyl]-5-[(5-fluoro-2-oxo-1-(N-ethylaminocarboxymethyl)-2,3-indolin-3-ylmethylene)]-2,4-di methyl-1***H***-pyrrolidine-3-carboxamide (Compound **5**); ***N***-[2-(diethylamino)ethyl]-5-[(5-fluoro-2-oxo-1-(N,N-dimethylaminocarboxymethyl)-2,3-indolin-3-ylmethylene)] -2,4-dimethyl-1***H***-pyrrolidine-3-carboxamide (Compound **6**); ***N***-[2-(diethylamino)ethyl]-5-[(5-fluoro-2-oxo-1-(t-butoxycarboxymethyl)-2,3-indolin-3-ylmethylene)]-2,4-dimeth yl-1***H***-pyrrolidine-3-carboxamide (Compound **7**); ***N***-[2-(diethylamino)ethyl]-5-[(5-fluoro-2-oxo-1-(ethoxycarboxymethyl)-2,3-indolin-3-ylmethylene)]-2,4-dimethyl -1***H***-pyrrolidine-3-carboxamide (Compound **8**); ***N***-[2-(diethylamino)ethyl]-5-[(5-fluoro-2-oxo-1-(cyclohexylcarboxymethyl)-2,3-indolin-3-ylmethylene)]-2,4-dim ethyl-1***H***-pyrrolidine-3-carboxamide (Compound **9**); ***N***-[2-(diethylamino)ethyl]-5-[(5-fluoro-2-oxo-1-(methylcarboxymethyl)-2,3-indolin-3-ylmethylene)]-2,4-dimethyl -1***H***-pyrrolidine-3-carboxamide (Compound **10**); ***N***-[2-(diethylamino)ethyl]-5-[(5-fluoro-2-oxo-1-(cyclopropylcarboxymethyl)-2,3-indolin-3-ylmethylene)]-2,4-dim ethyl-1***H***-pyrrolidine-3-carboxamide (Compound **11**); ***N***-[2-(diethylamino)ethyl]-5-[(5-fluoro-2-oxo-1-(isopropylcarboxymethyl)-2,3-indolin-3-ylmethylene)]-2,4-dimet hyl-1***H***-pyrrolidine-3-carboxamide (Compound **12**); or ***N***-[2-(diethylamino)ethyl]-5-[(5-fluoro-2-oxo-1-(p-methoxyphenylcarboxymethyl)-2,3-indolin-3-ylmethylene)]-2, 4-dimethyl-1***H***-pyrrolidine-3-carboxamide (Compound **13**).

In some embodiments, provided herein is a method for treating a cancer, comprising administering to a patient in need thereof an effective amount of any of the compounds or salts thereof or pharmaceutical compositions of the invention. In some embodiments, the cancer is metastatic renal cell carcinoma, gastrointestinal stromal tumor or liver

cancer.

In some embodiments, provided herein is a method for inhibiting tumor cell growth, comprising contacting the tumor cell with an effective amount of any of the compounds or salts thereof or pharmaceutical compositions of the invention. In some embodiments, the tumor cell is metastatic renal cell carcinoma cell, gastrointestinal stromal tumor cell or liver cancer cell.

In some embodiments, provided herein is a use of any of the compounds or salts thereof of the invention in preparation of a drug for treating a cancer. In some embodiments, the cancer is metastatic renal cell carcinoma, gastrointestinal stromal tumor or liver cancer.

In some embodiments, provided herein is a method for reducing toxicity of Sunitinib, comprising administering to a patient in need thereof an effective amount of any of the compounds or salts thereof or pharmaceutical compositions of the invention. In some embodiments, the cancer is metastatic renal cell carcinoma, gastrointestinal stromal tumor or liver cancer.

In the present context, the terms "therapeutically effective" and "effective amount" indicate that the materials or amount of material is effective to prevent, alleviate, or ameliorate one or more symptoms of a disease or medical condition, and/or to prolong the survival of the subject being treated.

The effective amounts of various compounds to be administered can be determined by standard procedures taking into account factors such as the compound $IC_{50}$, the biological half-life of the compound, the age, size, and weight of the subject, and the indication being treated. The importance of these and other factors are well known to those of ordinary skill in the art. Generally, a dose will be between about 0.01 and 50 mg/kg, preferably 0.1 and 20 mg/kg of the subject being treated. Multiple doses may be used.

The compounds of the invention may also be used in combination with other therapies for treating the same disease. Such combination use includes administration of the compounds and one or more other therapeutics at different times, or co-administration of the compound and one or more other therapies. In some embodiments, dosage may be modified for one or more of the compounds of the invention or other therapeutics used in combination, e.g., reduction in the amount dosed relative to a compound or therapy used alone, by methods well known to those of ordinary skill in the art.

It is understood that use in combination includes use with other therapies, drugs, medical procedures etc., where the other therapy or procedure may be administered at different times (e.g. within a short time, such as within hours (e.g. 1, 2, 3, 4-24 hours), or within a longer time (e.g. 1-2 days, 2-4 days, 4-7 days, 1-4 weeks)) than a compound of the present invention, or at the same time as a compound of the invention. Use in combination also includes use with a therapy or medical procedure that is administered once or infrequently, such as surgery, along with a compound of the invention administered within a short time or longer time before or after the other therapy or procedure. In some embodiments, the present invention provides for delivery of compounds of the invention and one or more other drug therapeutics delivered by a different route of administration or by the same route of administration. The use in combination for any route of administration includes delivery of compounds of the invention and one or more other drug therapeutics delivered by the same route of administration together in any formulation, including formulations where the two compounds are chemically linked in such a way that they maintain their therapeutic activity when administered. In one aspect, the other drug therapy may be co-administered with one or more compounds of the invention. Use in combination by co-administration includes administration of co-formulations or formulations of chemically joined compounds, or administration of two or more compounds in separate formulations within a short time of each other (e.g. within an hour, 2 hours, 3 hours, up to 24 hours), administered by the same or different routes. Co-administration of separate formulations includes co-administration by delivery via one device, for example the same inhalant device, the same syringe, etc., or administration from separate devices within a short time of each other. Co-formulations of compounds of the invention and one or more additional drug therapies delivered by the same route includes preparation of the materials together such that they can be administered by one device, including the separate compounds combined in one formulation, or compounds that are modified such that they are chemically joined, yet still maintain their biological activity. Such chemically joined compounds may have a linkage that is substantially maintained *in vivo,* or the linkage may break down *in vivo,* separating the two active components.

In some embodiments, the compound of the invention is selected from the group consisting of:

***N***-[2-(diethylamino)ethyl]-5-[(5-fluoro-2-oxo-1-(N,N-dimethylamino)methylcarboxymethyl-2,3-indolin-3-yl methylene)]-2,4-dimethyl-1***H***-pyrrolidine-3-carboxamide (Compound **A**);

***N***-[2-(diethylamino)ethyl]-5-[(5-fluoro-2-oxo-1-(t-butylcarboxymethyl)-2,3-indolin-3-ylmethylene)]-2,4-dimethyl-1***H***-pyrrolidine-3-carboxamide (Compound **1**);

***N***-[2-(diethylamino)ethyl]-5-[(5-fluoro-2-oxo-1-(phenylcarboxymethyl)-2,3-indolin-3-ylmethylene)]-2,4-dimethyl-1***H***-pyrrolidine-3-carboxamide (Compound **2**);

***N***-[2-(diethylamino)ethyl]-5-[(5-fluoro-2-oxo-1-(1-methylcyclobutylcarboxymethyl)-2,3-indolin-3-ylmethyl ene)]-2,4-dimethyl-1***H***-pyrrolidine-3-carboxamide (Compound **3**);

***N***-[2-(diethylamino)ethyl]-5-[(5-fluoro-2-oxo-1-(N-methylaminocarboxymethyl)-2,3-indolin-3-ylmethylene )]-2,4-dimethyl-1***H***-pyrrolidine-3-carboxamide (Compound **4**);

***N***-[2-(diethylamino)ethyl]-5-[(5-fluoro-2-oxo-1-(N-ethylaminocarboxymethyl)-2,3-indolin-3-ylmethylene)]-2,4-dimethyl-1***H***-pyrrolidine-3-carboxamide(Compound **5**);

***N***-[2-(diethylamino)ethyl]-5-[(5-fluoro-2-oxo-1-(N,N-dimethylaminocarboxymethyl)-2,3-indolin-3-ylmethylene)]-2,4-dimethyl-1***H***-pyrrolidine-3-carboxamide (Compound **6**);

***N***-[2-(diethylamino)ethyl]-5-[(5-fluoro-2-oxo-1-(t-butoxycarboxymethyl)-2,3-indolin-3-ylmethylene)]-2,4-dimethyl-1***H***-pyrrolidine-3-carboxamide (Compound **7**);

***N***-[2-(diethylamino)ethyl]-5-[(5-fluoro-2-oxo-1-(ethoxycarboxymethyl)-2,3-indolin-3-ylmethylene)]-2,4-dimethyl-1***H***-pyrrolidine-3-carboxamide (Compound **8**);

***N***-[2-(diethylamino)ethyl]-5-[(5-fluoro-2-oxo-1-(cyclohexylcarboxymethyl)-2,3-indolin-3-ylmethylene)]-2,4-dimethyl-1***H***-pyrrolidine-3-carboxamide (Compound **9**);

***N***-[2-(diethylamino)ethyl]-5-[(5-fluoro-2-oxo-1-(methylcarboxymethyl)-2,3-indolin-3-ylmethylene)]-2,4-dimethyl-1***H***-pyrrolidine-3-carboxamide (Compound **10**);

***N***-[2-(diethylamino)ethyl]-5-[(5-fluoro-2-oxo-1-(cyclopropylcarboxymethyl)-2,3-indolin-3-ylmethylene)]-2,4-dimethyl-1***H***-pyrrolidine-3-carboxamide (Compound **11**);

***N***-[2-(diethylamino)ethyl]-5-[(5-fluoro-2-oxo-1-(isopropylcarboxymethyl)-2,3-indolin-3-ylmethylene)]-2,4-dimethyl-1***H***-pyrrolidine-3-carboxamide (Compound **12**); and

***N***-[2-(diethylamino)ethyl]-5-[(5-fluoro-2-oxo-1-(p-methoxyphenylcarboxymethyl)-2,3-indolin-3-ylmethylene)]-2,4-dimethyl-1***H***-pyrrolidine-3-carboxamide (Compound **13**).

**Pharmaceutical Composition**

Also provided herein is a pharmaceutical composition comprising a compound of formula I or a pharmaceutically acceptable salt thereof. The compound of formula I comprised in the pharmaceutical composition has a weight ratio of 0.1-99.9% in the composition, and a pharmaceutically acceptable carrier has a weight ratio of 0.1-99.9% in the composition. The pharmaceutical composition exists as a formulation suitable for pharmaceutical use. Pharmaceutical formulations include tablets, capsules, granules, pills, powders, pastes, suspensions, injections, powder injections, suppositories, creams, drops or patches. The tablets can be sugar-coated tablets, film-coated tablets, enteric-coated tablets or sustained-release tablets; the capsules can be hard capsules, soft capsules, sustained-release capsules; and the powder injections can be freeze-dried powder injections.

The pharmaceutical composition of the invention, as a formulation, has an effective amount of 0.1-1000 mg of a compound of the invention in each formulation. Said each formulation refers to each formulation unit, such as each tablet, each capsule, and also refers to each dosage, such as 100 mg each dosage.

Solid carriers can be used in the preparation of the pharmaceutical composition of the invention into solid or semi-solid pharmaceutical formulations such as powders, tablets, dispersible powders, capsules, cachets, suppositories and ointments. Solid carriers that can be used are preferably selected from one or more substances in diluents, flavoring agents, solubilizers, lubricants, suspending agents, binders, expanders and the like, or can be encapsulated substances. In powder formulations, the carrier may comprise 5-70% of micronized active ingredient. Suitable solid carriers include magnesium carbonate, magnesium stearate, talc, sucrose, lactose, pectin, dextrin, starch, gelatin, methylcellulose, sodium carboxymethyl cellulose, low boiling wax, cocoa butter and the like. Tablets, powders, cachets and capsules and the like represents the most favorable oral solid formulations since they are easy to administer.

Liquid formulations of the present invention includes solutions, suspensions and emulsions. For example, injectable formulations for parenteral administration can be in the form of water or water-propylene glycol solution, whose isotonicity, pH and the like can be adjusted to be suitable for physiological conditions of living body. Liquid formulations can also be prepared into the form of solution in polyethylene glycol or aqueous solution. Oral aqueous solutions can

be prepared by dissolving active ingredients in water, followed by adding suitable coloring agents, flavoring agents, stabilizers and thickening agents. Water suspensions suitable for oral administration can be prepared by dispersing micronized active ingredients in a sticky substance, such as natural and synthetic gums, methylcellulose, sodium carboxymethyl cellulose, and other known suspending agents.

In order to provide ease of administration and uniformity of dosage, it is particularly advantageous that the above pharmaceutical formulations are prepared into dosage unit forms. A dosage unit form of a formulation refers to a physically separable unit that is suitable for single dose, each unit comprising a calculated predetermined amount of an active ingredient that can provide expected treatment effect. Such dosage unit form can be a package form, such as a tablet, a capsule or a powder packed in a tubule or vial, or an ointment, gel or cream packed in a tube or bottle.

Although the amount of an active ingredient comprised in a dosage unit form may vary, it is generally adjusted to be in the range of 1-800 mg, depending on the effect of the chosen active ingredient.

When the active compound of formula (I) of the invention is used as an anti-tumor drug, its administration dosage may vary with the requirement of patient, severity of the condition to be treated, and the compound that is chosen.

Preferable dosage for a certain condition may be determined by a skilled person in the art according to conventional methods. In general, dosage in the start of treatment may be lower than optimal dosage of an active ingredient, and then it is gradually increased until optimal treatment effect is reached. For the needs of treatment, a total daily dosage may be provided in one administration, or in several administrations.

## BRIEF DESCRIPTION OF THE DRAWINGS

Figure 1 shows an illustration of pharmacokinetic study for intravenous administration of Sunitinib and Compound **A** in mice.

Figure 2 shows an illustration of concentration-time curve for intravenous administration of Sunitinib and Compounds **1** and **2** in mice, wherein (a) heart; (b) liver; (c) spleen; (d) lung; (e) kidney; (f) plasma.

Figure 3 shows an illustration of concentration-time curve for intragastric administration of Sunitinib and Compounds **1** and **2** in mice, wherein (a) heart; (b) plasma.

Figure 4 shows an illustration of comparison for dephosphorylation effect of Sunitinib and Compounds **1** and **2** for pSTAT3.

Figure 5 shows an illustration of cytotoxicity experiments of Sunitinib and Compounds **1** and **2** for different cell lines, wherein (a) A549; (b) NCI-H460; (c) 786-0; (d) Caki-1; (e) HT-29; (f) MCF-7.

Figure 6 shows an illustration of acute toxicity test for oral administration of Compound **2** and Sunitinib in mice, wherein (a) 28 mg/kg; (b) 70 mg/kg; (c) 175 mg/kg; (d) 50 mg/kg; (e) 100 mg/kg; (f) 200 mg/kg.

Figure 7 shows an illustration of comparison for average tumor size in mice with cancer cell xenograft after subcutaneous injection of Compounds **1** and **2** and Sunitinib, wherein (a) 1.5 mg/kg; (b) 7.5 mg/kg; (c) 30 mg/kg.

Figure 8 shows an illustration of comparison for average tumor size in mice with cancer cell xenograft after oral administration of Compounds **1** and **2** and Sunitinib, wherein (a) 5 mg/kg; (b) 15 mg/kg; (c) 45 mg/kg.

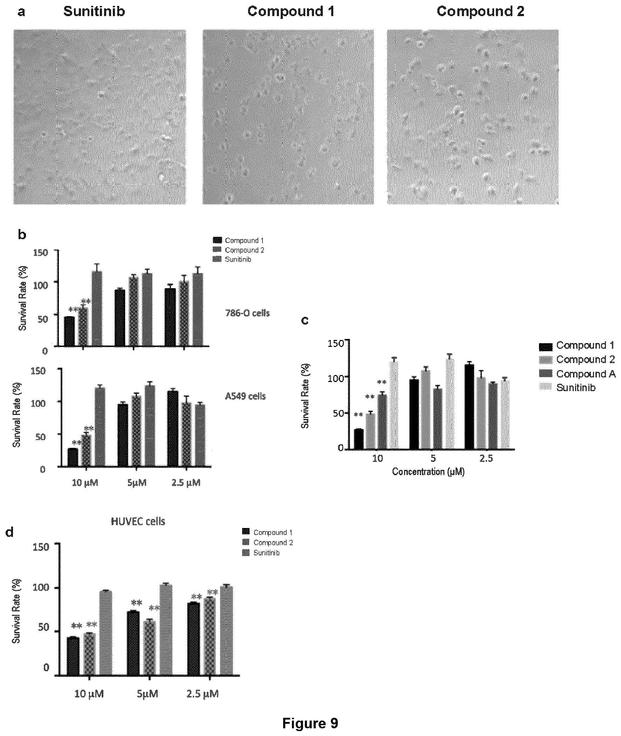

Figure **9** shows effects of Compounds **1** and **2** on cell morphology and survival rate, wherein (a) effect on cell morphology of Sunitinib and Compounds **1** and **2**; (b) effect on cell survival rate of Sunitinib and Compounds **1** and **2** in different concentrations and co-cultured with 786-0 cell or A549 cell for 8 hours; (c) effect on cell survival rate of Sunitinib and Compounds **A**, **1** and **2** in different concentrations and co-cultured with A549 cell for 8 hours; (d) effect on cell survival rate of Sunitinib and Compounds **1** and **2** in different concentrations and co-cultured with HUVEC cell for 8 hours.

Figure **10** shows effects of Sunitinib and Compound **2** on key biochemical indicators, wherein (a) Sunitinib significantly increases aspartate transaminase (AST), alanine aminotransferase (ALT) and cholinesterase (CHE); (b) Sunitinib increases blood uric acid and blood urea nitrogen levels, but decreases blood sugar; (c) effects of Sunitinib and Compound **2** on lactate dehydrogenase (LDH), creatine kinase (CK), creatine kinase-MB (CKMB) indicators.

Figure **11** shows effects of compounds of the invention on survival rate of A549 human non-small cell lung cancer cell line after a short-term treatment (8h) to it, wherein (**a**) to (**h**) represents Compounds **6** to **13** respectively.

Figure **12** shows effects of compounds of the invention on survival rate of A549 human non-small cell lung cancer cell line after a short-term treatment (8h) to it followed by removing the compounds and recovery for 24 hours, wherein (**a**) to (**h**) represents Compounds **6** to **13** respectively.

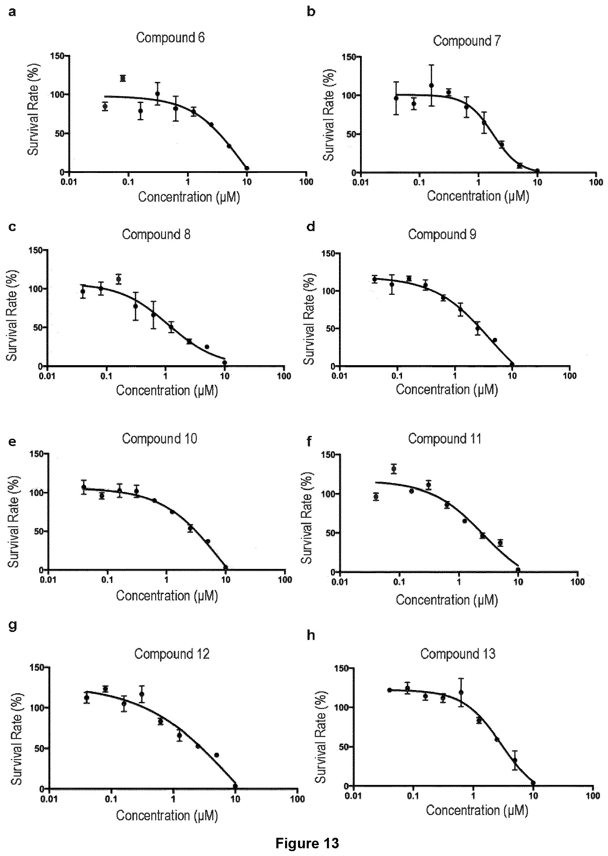

Figure **13** shows effects of compounds of the invention on survival rate of A549 human non-small cell lung cancer cell line after a long-term treatment (72h) to it, wherein (**a**) to (**h**) represents Compounds **6** to **13** respectively.

## DETAILED DESCRIPTION

Prodrugs refer to compounds that have no or very low in vivo activity after chemical structural alteration, which release the original drugs to provide pharmacological effects after enzymatic or nonenzymatic processes in vivo.

In general, the prodrugs are prepared by modifying drugs using "lipophilic", "hydrophilic", "active transport" and "double drugs" strategies, with purposes to increase bioavailability and stability, reduce toxic side effect, improve long-term effect, cover up discomfortable smell, and so on. Fosamprenavir, one of the most successful prodrugs, is a "hydrophilic" phosphate ester precursor of ampunavir, and is now widely used in AIDS treatment.

Basing on Sunitinib, in order to provide it with more excellent features, a number of prodrugs of Sunitinib are designed by the inventors by alterations to the structure of Sunitinib using related prodrug design principles. The inventors was surprised to find that the compounds of the invention have a liver concentration much higher than Sunitinib, provides a greater lethality to liver cancer, and at the same time reduces systemic toxicity, especially cardiac toxicity, and reduces adverse effects.

It is difficult to predict the effect of a certain substituent in the preparation of a prodrug using Sunitinib as the parent compound. It is well known in the art that there are thousands of substituents that can be used for a prodrug, and that it is extremely difficult to screen out an appropriate and applicable substituent.

Provided herein is a Sunitinib prodrug having formula I

formula (I)

wherein:

$R_{12}$, $R_{13}$ are selected from the group consisting of H, $C_1$-$C_6$alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl and 5-15 membered heteroaryl;

$R_{14}$ is selected from the group consisting of R', OR', SR' and $N(R')_2$; and

R' is selected from the group consisting of H, $C_1$-$C_6$alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl, 5-15 membered heteroaryl, hydroxyl $C_1$-$C_6$ alkyl, carboxyl $C_1$-$C_6$ alkyl, $C_1$-$C_6$ alkyl amido and phosphate group.

In some embodiments, $R_{12}$, $R_{13}$ are H, and $R_{14}$ is selected from the group consisting of N,N-dimethylaminomethyl, t-butyl, phenyl, p-fluorophenyl, biphenyl, dimethylamino, cyclopropyl, methyl, ethyl, propyl, isopropyl, butyl, isobutyl, cyclobutyl, pentyl, isopentyl, neopentyl, cyclopentyl, 1-methylcyclobutyl, N-methylamino, N-ethylamino, N,N-methylethylamino, n-hexyl, cyclohexyl, methylthio, ethylthio, propylthio, isopropylthio, cyclopropylthio, butylthio, isobutylthio, cyclobutylthio, methoxy, ethoxy, propoxy, isopropoxy, butoxy, cyclobutoxy, pentylamino, pentylthio, pentyloxy, isopentylamino, neopentylamino, t-butylamino, cyclopentylamino, cyclopentylthio, cyclopentyloxy, cyclohexylamino, cyclohexylthio, cyclohexyloxy, p-methoxyphenyl, p-chlorophenyl and o-fluorophenyl.

In some embodiments, $R_{12}$, $R_{13}$ are both H, and $R_{14}$ is t-butyl, phenyl or t-butoxy.

**Example 1.** Preparation of N-[2-(diethylamino)ethyl]-5-[(5-fluoro-2-oxo-1-hydroxymethyl-2,3-indolin-3-ylmethylene)]-2,4-dimethyl-1 H-pyrrolidine-3-carboxamide (Compound X)

HCHO

TEA/DMF

At 0°C, to a solution of Sunitinib (1.25mmol, 0.5 g) in DMF (14 ml) was added formaldehyde (30%, 405mg, 5mmol) and triethylamine (506 mg, 5 mmol). The mixture was stirred overnight and cooled to 0°C. Water was added to the mixture while stirring to form a yellow solid precipitate. The solid was filtered, washed and dried in air for days to give 400 mg product (compound X, yield 75%). $^1$H NMR (400 MHz, DMSO-d6): 13.56 (s, 1H), 7.84 (d, 1H, J=9.2 Hz), 7.77 (s, 1H), 7.49 (t, 1H, J=5.2 Hz), 7.15-7.00 (m, 2H), 6.35 (s, 1H), 5.24 (s, 2H), 3.31-3.26 (m, 2H), 2.56-2.52 (m, 6H), 2.49-2.44 (m, 6H), 0.98 (t, 6H, J=7.2 Hz).

**Example 2.** Preparation of *N*-[2-(diethylamino)ethyl]-5-[(5-fluoro-2-oxo-1-(N,N-dimethylamino)methylcarboxymethyl-2,3-indolin-3-ylmethylene)]-2,4-dimethyl-1 H-pyrrolidine-3-carboxamide (Compound A)

EDC/DMAP
DMF, Py

N,N-dimethylglycine

The compound X (437 mg, 1.02 mmol) as obtained in Example 1 was dissolved in 5 mL anhydrous DMF and 2 mL pyridine (Py). To the mixture was added N,N-dimethylaminopyridine (12 mg, 0.1 mmol), N,N-dimethylglycine (206 mg, 2 mmol) and EDC (384 mg, 2 mmol), followed by stirring at room temperature for 2 hours. The solvents were removed under reduced pressure and the remaining product was extracted by DCM, washed by saline and dried on anhydrous $Na_2SO_4$. The product was purified by silica gel chromatography with DCM/MeoH (0.1% Et3N was added in the DCM). The fraction was collected and dried under vacuum to give 50 mg orange solid (Compound A, yield 10%). $^1$H NMR (400 MHz, DMSO-d6): 13.25 (s, 1H), 7.86 (dd, 1H, J=7.2, 2 Hz), 7.81 (s, 1H), 7.50 (t, 1H, J=4.4 Hz), 7.19(dd, 1H, J=7.2, 3.2Hz), 7.05 (td, 1H, J=7.2, 2Hz), 5.93 (s, 2H), 3.31-3.27 (m, 4H), 3.19(s, 2H); 2.56-2.44 (m, 10H), 2.21(s, 6H), 0.98 (t, 6H, J=7.2 Hz).

**Example 3.** Preparation of ***N*-[2**-(diethylamino)ethyl**]-5-[(5-**fluoro-2-oxo**-1-(*t*-butyl**carboxymethyl**)-2,3-**indolin**-3-**ylmethylene**)]-2,4-**dimethyl**-1*H*-**pyrrolidine-3-carboxamide (Compound **1**)

To the solution of compound X (1.65g, 3.86 mmol, 1eq.) in pyridine (50 mL) was added trimethylacetic anhydride (2.87g, 15.42 mmol, 4 eq.) and DMAP (50 mg). The mixture was stirred under room temperature overnight and then concentrated to dry. The residue was added with saturated $NaHCO_3$ solution (20 mL) and extracted with EtOAc (2 x 20mL). The combined organic phases were dried (anhydrous $Na_2SO_4$), filtered, concentrated, purified on a silica gel column and eluted with DCM:methanol (10:1) to obtain compound 1 as a yellow solid (1g, yield 51%, confirmed by $^1H$ NMR and HPLC, batch # MC09179-056-15S). $^1H$ NMR (400 MHz, DMSO-d6) δ13.26 (br s, 1 H), 8.87 (dd, J1=9.6 Hz, J2=2.4 Hz, 1 H), 7.81 (s, 1 H), 7.54-7.52 (m, 1 H), 7.17-7.05 (m, 2H), 5.90 (s, 2H), 3.32-3.27 (m, 4H), 2.58-2.45 (m, 10H), 1.11 (s, 9H), 0.98 (t, J=7.2 Hz, 6H); HPLC purity: 96.27% (254 nm).

**Example 4.** Preparation of ***N*-[2-(**diethylamino)ethyl**]-5-[(5-**fluoro-2-oxo**-1-(phenyl**carboxymethyl**)-2,3-**indolin**-3-**ylmethylene**)]-2,4-**dimeth yl**-1*H*-**pyrrolidine-3-carboxamide (Compound **2**)

To the solution of compound X (1.65g, 3.86 mmol, 1eq.) in pyridine (50 mL) was added $(PhCO)_2O$ solution (1.7g, 7.7mmol, 2 eq.) and DMAP (50 mg). The mixture was stirred under room temperature overnight and then concentrated to dry. The residue was added with saturated $NaHCO_3$ solution (20 mL) and extracted with EtOAc (2 x 20mL). The combined organic phases were dried (anhydrous $Na_2SO_4$), filtered, concentrated, purified on a silica gel column and eluted with DCM:methanol (10:1) to obtain compound 2 as a yellow solid (1.2g, yield 58%, confirmed by $^1H$ NMR and HPLC, batch # MC09179-055-11S). $^1H$ NMR (400 MHz, DMSO-d6) δ13.28 (br s, 1 H), 7.95-7.84 (m, 4 H), 7.67 (t, J=7.2 Hz, 1 H), 7.54-7.50 (m, 3H), 7.34-7.31 (m, 1H), 7.10-7.05 (m, 1H), 6.18 (s, 2H), 3.32-3.27 (m, 4H), 2.57-2.45 (m, 10H), 0.98 (t, J=7.2 Hz, 6H); HPLC purity: 96.86% (254 nm).

**Example 5.** Preparation of ***N*-[2-(**diethylamino)ethyl**]-5-[(5-**fluoro-2-oxo**-1-(**N,N-dimethylaminocarboxymethyl**)-2,3-**indolin**-3-**ylmethylene)**] -2,4-**dimethyl**-1*H*-**pyrrolidine-3-carboxamide (Compound **6**)

1) CDI; DMF

2) TEA, dimethylamine HCl

MW: 428.22

MW: 499.26

To the solution of compound X (150mg, 0.35mmol) in anhydrous DMF (2mL) was added CDI (62.4mg, 0.385 mmol, 1.1 eq). The mixture was stirred at room temperature for 30 min before addition with triethylamine (56.7 mg, 0.56mmol) and dimethylamine hydrochloride (45.67mg, 0.56mmol). The reactants were stirred at room temperature for 1 hour before addition with 5 mL water and extracted with $CH_2Cl_2$ (3 X 2 mL). The combined organic phases were dried (anhydrous $Na_2SO_4$), filtered, concentrated, purified by TLC and eluted with DCM:MeOH (10:1) to obtain compound 6 as a yellow solid (20 mg, yield 45%, Lot #: MC10188-20-6, confirmed by $^1H$ NMR and MS, HPLC: 98%, 254nm). $^1H$ NMR (400 MHz, CDCl3): 13.37 (1 H, s), 11.58 (1 H, br s), 7.92 (s, 1 H), 7.36 (s, 1H), 7.14-7.18 (m, 2H), 6.88-6.93 (m, 1H), 5.91 (s, 2H), 3.97-3.91 (m, 2H), 3.25-3.31 (m, 2H), 3.15-3.20 (m, 2H), 2.99-2.30 (m, 6H), 2.64 (s, 3H), 2.57 (s, 3H), 1.57-1.59 (br s, 4H), 1.42-1.45 (m, 6H).

**Example 6.** Preparation of ***N*-[2-(**diethylamino)ethyl**]-5-[(5-**fluoro-2-oxo**-1-(*t*-**butoxycarboxymethyl**)-2,3-**indolin**-3-**ylmethylene**)]-2,4-**dimet hyl**-1*H*-**pyrrolidine-3-carboxamide (Compound **7**)

To the solution of compound X (100 mg, 0.26mmol) in anhydrous Py (3 mL) was added DMAP (3.16mg, 0.026 mmol, 0.1 eq.) and SM 3 (173 mg, 1.04 mmol). The mixture was stirred at room temperature for 16 hours before concentration. The residue was added with saturated $NaHCO_3$ solution and extracted with EA. The combined organic phases were dried, filtered, concentrated, purified on a silica gel column and eluted with DCM:MeOH (10:1) to obtain compound 7 as a yellow solid (80 mg, yield 58.4%, Lot#: MC10188-004-6, confirmed by $^1$H NMR and MS, HPLC:95.6%, 254 nm).$^1$H NMR (400 MHz, $CDCl_3$-$d_6$): δ ppm 13.31 (bs, 1H), 7.38 (s, 1H), 7.21-7.19 (m, 1H), 7.09-7.06 (m, 1H), 6.94-6.88 (m, 1H), 6.47 (bs, 1H), 5.88 (s, 2H), 3.49-3.48 (m, 2H), 2.67 (m, 2H), 2.60-2.57(m, 7H), 2.51-2.39 (m, 3H), 1.11 (s, 9H), 1.04(t, J=7 Hz, 6H).

**Example 7.** Preparation of ***N*-[2-**(diethylamino)ethyl**]-5-[(5-**fluoro-2-oxo**-1-**(ethoxylcarboxymethyl**)-2,3-**indolin**-3-**ylmethylene**)]-2,4-**dimeth yl**-1*H*-**pyrrolidine-3-carboxamide (Compound **8**)

To the solution of compound X (100 mg, 0.26mmol) in anhydrous Py (3 mL) was added DMAP (3.16mg, 0.026 mmol, 0.1 eq.) and SM 2 (169 mg, 1.04 mmol). The mixture was stirred at room temperature for 16 hours before concentration. The residue was added with saturated $NaHCO_3$ solution and extracted with EA. The combined organic phases were dried, filtered, concentrated, purified on a silica gel column and eluted with DCM:MeOH (10:1) to obtain compound 8 as a yellow solid (70 mg, yield 53.8%, Lot#: MC10188-002-6, confirmed by $^1$H NMR and MS, HPLC:98.7%, 254 nm). $^1$H NMR(400 MHz, $CDCl_3$-$d_6$): δ ppm 13.29 (bs, 1 H), 7.38 (s, 1 H),7.22-7.19 (m, 1 H), 7.09-7.06 (m, 1 H), 6.94-6.91 (m, 1 H), 6.47 (bs, 1 H), 5.94 (s, 2H), 4.26-4.21 (m, 2H), 3.51-3.47 (m, 2H), 2.67-2.63(m, 2H), 2.60-2.57 (m, 7H), 2.55-2.51 (m, 3H), 1.30(t, J=7 Hz, 3H), 1.4(t, J=7 Hz, 6H).

**Example 8.** Preparation of ***N*-[2-(**diethylamino)ethyl**]-5-[(5-**fluoro-2-oxo**-1-(**cyclohexylcarboxymethyl**)-2,3-**indolin**-3-**ylmethylene**)]-2,4-**dim ethyl**-1*H*-**pyrrolidine-3-carboxamide (Compound **9**)

DMAP/Py, rt, 16 h

SM 4

MW: 428.22

To the solution of compound X (100 mg, 0.26mmol) in anhydrous Py (3 mL) was added DMAP (3.16mg, 0.026 mmol, 0.1 eq.) and SM 4 (248 mg, 1.04 mmol). The mixture was stirred at room temperature for 16 hours before concentration. The residue was added with saturated $NaHCO_3$ solution and extracted with EA. The combined organic phases were dried, filtered, concentrated, purified on a silica gel column and eluted with DCM:MeOH (10:1) to obtain compound 9 as a yellow solid (60 mg, yield 43%, Lot#: MC10188-006-6, confirmed by $^1$H NMR and MS, HPLC:99%, 254 nm).$^1$H NMR(400 MHz, $CDCl_3$-$d_6$): δ ppm 13.41 (bs, 1H), 7.39 (s, 1 H), 7.22-7.18 (m, 1 H), 7.04-7.01 (m, 1 H), 6.93-6.89 (m, 1 H), 6.47 (bs, 1 H), 5.92 (s, 2H), 3.49-3.48 (m, 2H), 2.67-2.61 (m, 2H), 2.57-2.52(m, 7H), 2.47-2.45 (m, 3H), 2.36-2.35(m, 1H),1.89-1.85(m, 2 H), 1.67-1.62(m, 2 H), 1.43-1.40( m, 3 H), 1.26-1.20(m, 3H), 1.04(m, 6H)

**Example 9.** Preparation of ***N*-[2-(**diethylamino)ethyl**]-5-[(**5-fluoro-2-oxo**-1-(**methylcarboxymethyl**)-2,3-**indolin**-3-**ylmethylene**)]-2,4-**dimethy **l-1*H*-**pyrrolidine-3-carboxamide (Compound **10**)

DMAP/Py, rt, 16 h

SM 1

MW: 428.22

MW: 470.23

To the solution of compound X (100 mg, 0.26mmol) in anhydrous Py (3 mL) was added DMAP (3.16mg, 0.026 mmol, 0.1 eq.) and SM 1 (106 mg, 1.04 mmol). The mixture was stirred at room temperature for 16 hours before concentration. The residue was added with saturated $NaHCO_3$ solution and extracted with EA. The combined organic phases were dried, filtered, concentrated, purified on a silica gel column and eluted with DCM:MeOH (10:1) to obtain compound 10 as a yellow solid (60 mg, yield 49%, Lot#: MC10188-005-6, confirmed by $^1$H NMR and MS, HPLC:96.5%, 254 nm). $^1$H NMR(400 MHz, $CDCl_3$-$d_6$): δ ppm 13.32 (bs, 1 H), 7.39 (s, 1H),7.21-7.19 (m, 1H), 7.07-7.05 (m, 1H), 6.94-6.89 (m, 1H), 6.60 (bs, 1H), 5.91 (s, 2H), 3.53-3.52 (m, 2H), 2.71-2.67 (m, 2H), 2.63-2.52(m, 7H), 2.45-2.44 (m, 3H), 2.09 (s, 3H), 1.07(s, 6H).

**Example 10.** Preparation of ***N*-[2-(**diethylamino)ethyl**]-5-[(5-**fluoro-2-oxo-**1-(**cyclopropylcarboxymethyl**)-2,3-**indolin-**3-**ylmethylene**)]-2,4-**di methyl-1***H***-pyrrolidine-3-carboxamide (Compound **11**)

MW: 428.22 + RI MW: 154.06 → (DMAP, pyridine; rt, overnight) MW: 496.25

To the solution of compound X (200 mg, 0.467 mmol) in pyridine (5 mL) was added DMAP (5 mg), and then compound RI/pyridine (288 mg, 1.87 mmol, 4.0 eq.) in ice bath.The mixture was stirred at room temperature overnight before concentration. The residue was purified on a silica gel column and eluted with DCM:MeOH (50:1) to obtain compound 11 as a yellow solid (138 mg, yield 60%, Lot#: MC09163-034-P1, confirmed by $^1$H NMR and MS, HPLC:99%, 254 nm). $^1$H NMR (400 MHz, DMSO-d6): 13.26 (s, 1H), 7.88 (dd, 1H, *J*=11.6 Hz), 7.82 (s, 1H), 7.52 (t, 1H, *J*=10.8 Hz), 7.22~7.16 (m, 1H), 7.10~7.02 (m, 1H), 5.91 (s, 2H), 3.35~3.28 (m, 2H), 2.57~2.53 (m, 4H), 2.52~2.51 (m, 8H), 1.69~1.62 (m, 1H), 0.99 (t, 6H, J=14 Hz), 0.95~0.85 (m, 4H).

**Example 11.** Preparation of ***N*-[2-(**diethylamino)ethyl**]-5-[(5-**fluoro-2-oxo-**1-(**isopropylcarboxymethyl**)-2,3-**indolin-**3-**ylmethylene**)]-2,4-**dimet hyl-**1*H*-**pyrrolidine-3-carboxamide (Compound **12**)

MW: 428.22 + RJ MW: 158.09 → (DMAP, pyridine; rt, overnight) MW: 498.26

To the solution of compound X (200 mg, 0.47 mmol) in pyridine (5 mL) was added DMAP (5 mg), and then compound RJ/pyridine (295 mg, 1.87 mmol, 4.0 eq.) in ice bath. The mixture was stirred at room temperature overnight before concentration. The residue was purified on a silica gel column and eluted with DCM:MeOH (50:1) to obtain compound 12 as a yellow solid (135 mg, yield 58%, Lot#: MC09163-040-P, confirmed by $^1$H NMR and MS, HPLC:99%, 254 nm). $^1$H NMR (400 MHz, DMSO-d6): 13.26 (s, 1 H), 7.88 (dd, 1H, *J*=8.4 Hz), 7.81 (s, 1H), 7.54~7.52 (m, 1H), 7.19~7.15 (m, 1H), 7.05 (t, 1H,J=17.6 Hz), 5.89 (s, 2H), 2.58~2.56 (m, 8H), 2.48~2.44 (m, 6H), 1.06 (d, 7H, J=6.8 Hz), 1.0~0.97 (m, 6H).

**Example 12.** Preparation of ***N*-[2-(**diethylamino)ethyl**]-5-[(5-**fluoro-2-oxo-**1-(***p*-methoxyphenylcarboxymethyl**)-2,3-**indolin-**3-**ylmethylene**)]-2 ,4-**dimethyl-**1*H*-**pyrrolidine-3-carboxamide (Compound **13**)

MW: 428.22 + RL MW: 286.08 → DMAP, pyridine, rt, overnight → MW: 562.26

To the solution of compound X (200 mg, 0.47 mmol) in pyridine (5 mL) was added DMAP (5 mg), and then compound RL/pyridine (534 mg, 1.87 mmol, 4.0 eq.) in ice bath. The mixture was stirred at room temperature overnight before concentration. The residue was purified on a silica gel column and eluted with DCM:MeOH (50:1) to obtain compound 13 as a yellow solid (120 mg, yield 45%, Lot#: MC09163-041-P1, confirmed by $^{1}$H NMR and MS, HPLC:98.9%, 254 nm). $^{1}$H NMR (400 MHz, DMSO-d6): 13.29 (s, 1 H), 7.89 (d, 3H, $J$=8.8 Hz), 7.83 (s, 1H), 7.55 (s, 1H), 7.33~7.29 (m, 1H), 7.09~7.02 (m, 3H), 6.13 (s, 2H), 3.83 (s, 3H), 3.0~2.5 (m, 7H), 2.49~2.45 (m, 7H), 0.84 (s, 6H).

**Example 13.** Pharmacokinetics and Stability Study of Compound A

Pharmacokinetics

*Methods*: Sunitinib and Compound A were dissolved in malate solution and adjusted to about pH 7. 5.14 mg/kg Sunitinib malate was injected through caudal vein of mice in control group (n=6) and 13 mg/kg compound A was injected through caudal vein of mice in sample A group (n=6). For each group, 0.083h, 0.25h, 0.5h, 1h, 3h, 8h post injection, blood was taken by eyeball removal and the mice were sacrificed by cervical dislocation. Tissues were perfused with normal saline and livers and kidneys were removed and cleaned with normal saline. The blood samples were centrifuged to obtain supernatants and stored at -20°C. 50 µL plasma sample and 50 µL internal standard (clozapine) working solution was vortexed with 300µL MeOH and then centrifuged. 150 µL upper organic phase was obtained and mixed with 150 µL water, followed by centrifugation. 10 µL supernatant was sampled for analysis. The tissue samples were weighted and mixed homogeneously with 10 times weight of water. Since the samples were 10-time diluted, the concentration of the tissue would be 10 times the results determined. The heart tissue was 5-time diluted. 100 µL homogenate was mixed with 50 µL internal standard (clozapine) working solution and 300µL MeOH and then centrifuged. 150 µL upper organic phase was obtained and mixed with 150 µL water, followed by centrifugation. 10 µL supernatant was sampled for analysis.

*Results and Analysis*

$$\text{Relative Exposure (RE)} = \text{AUCi sample}/\text{AUCi control}$$

AUCi - area under curve for the $i^{th}$ organ or tissue; RE>1 indicates targeting at the organ or tissue, with larger RE indicating greater targeting; RE≤1 indicates non-targeting.

Relative Total Exposure (RTE)

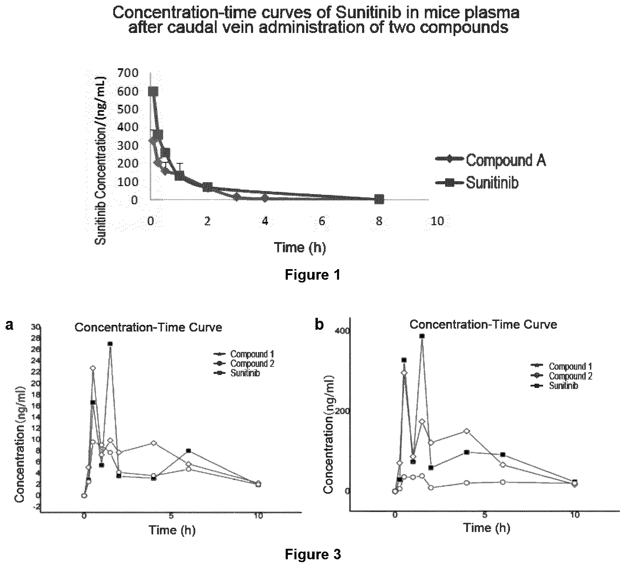

RTE refers to the multiple of the percent of drug content in a tissue of sample group with respect to total drug amount in the body vs. that in a control group. Positive RTE values indicate enhanced targeting effects and the more positive the more the multiple of enhancement and the more the enhanced targeting. In contrast, negative RTE values indicate reduced targeting effects. Value 0 indicate no influence to the tissue. Results were shown in Fig. 1 and Tables 1 and 2.

Table 1. Distribution of Drug in Organs

| | $t_{1/2}$/h | | $AUC_{0\rightarrow\infty}$/(h*ng/mL) | | Cl(mL/h) | | V/mL | | $C_{max}$(ng/mL) | |
|---|---|---|---|---|---|---|---|---|---|---|
| | A | S | A | S | A | S | A | S | A | S |
| Heart | 1.1 | 2.2 | 11.6 | 70.9 | 6856 | 1043 | 10921 | 3331 | 6.7 | 50.4 |
| Liver | 1.8 | 2.2 | 72.8 | 207.0 | 1044 | 357 | 2777 | 1152 | 59.9 | 109.2 |
| Spleen | 1.4 | 1.7 | 59.6 | 263.1 | 1313 | 291 | 2581 | 694 | 24.1 | 75.3 |
| Lung | 1.5 | 1.5 | 79.7 | 392.3 | 977 | 198 | 2059 | 443 | 35.9 | 210.0 |
| Kidney | 1.1 | 1.5 | 42.4 | 169.2 | 1867 | 461 | 2928 | 991 | 20.1 | 80.2 |
| Plasma | 1.1 | 1.2 | 71.2 | 316.8 | 1822 | 446 | 1113 | 249 | 58.2 | 225.6 |

Table 2. Parameters of Targeting

| | RE | RTE |
|---|---|---|
| Heart | 0.16 | -0.31 |
| Liver | 0.35 | 0.48 |
| Spleen | 0.23 | -0.05 |
| Lung | 0.20 | -0.15 |
| Kidney | 0.25 | 0.06 |
| Plasma | 0.22 | -0.05 |

The pharmacokinetic parameters were calculated by Winnonlin software. Since more than 1 animals were used for pharmacokinetics study, mean values of concentration-time curves were obtained before introducing into the software for calculation.

As the results showed, in terms of clearance, Compound A (sample A) had clearance in heart, liver, spleen, lung and kidney that was 7, 3, 5, 5, 4, and 4 times than that in Sunitinib group (S, Sunitinib). The clearance in all tested organs were significantly increased. The multiple of increase in liver was least, possibly because some metabolites of Compound A (sample A) induced CPY metabolic enzymes or interfered clearance path such that the clearance of Sunitinib was enhanced, leading to the decrease of Sunitinib exposure value in sample A group.

Compound A is a prodrug which is converted into Sunitinib in the process of reaching respective organs. It is possible that the particular structure of Compound A leads to its targeting distribution. Therefore, its distribution in liver is increased even in the case of accelerated metabolism.

In terms of RTE, kidney had a RTE of approx. 0, plasma and other organs had a RTE less than 1, and only liver's RTE (0.48) significantly higher than 0, indicating Sunitinib distribution in Compound A group was decreased in all organs except for liver. Therefore, Compound A could promote the liver distribution of Sunitinib.

In view of the above, although Compound A was not able to elevate the absolute exposure of Sunitinib in all organs and tissues, it enhanced the distribution proportion of Sunitinib in liver (i.e., enhanced targeting) and decreased its distribution in other organs. In the event that neither Compound A (sample A) nor its metabolite was toxic, Compound A could reduce the toxicity of Sunitinib in non-targeted organs compared Sunitinib.

**Example 14.** Comparison Studies among Compounds 1, 2 and Sunitinib

Pharmacokinetics

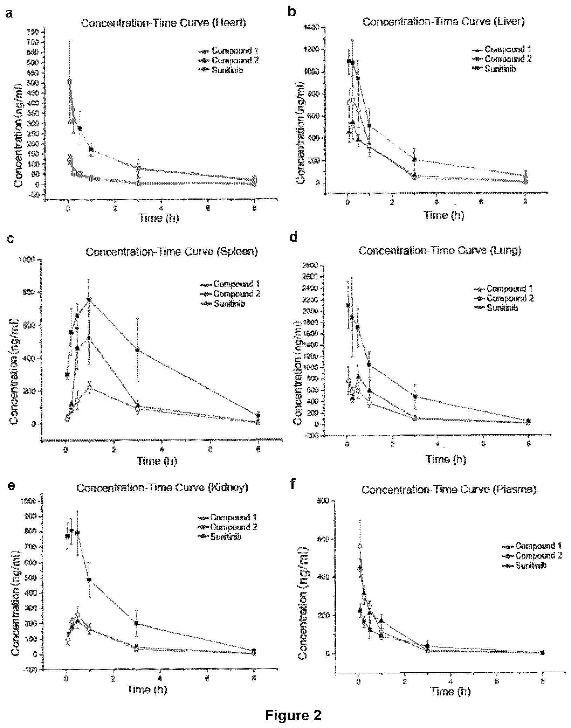

*Methods*: Sunitinib, Compound 1, and Compound 2 were dissolved in malate solution and adjusted to pH 7. 5.14 mg/kg Sunitinib malate, 5.13mg/kg Compound 1, and 5.32mg/kg Compound 2 was injected through caudal vein of mice, respectively (n=36 for each group). 0.083h, 0.25h, 0.5h, 1h, 3h, 8h post injection, blood was taken by eyeball removal and the mice were sacrificed by cervical dislocation. Tissues were perfused with normal saline and livers and kidneys were removed and cleaned with normal saline. The blood samples were centrifuged to obtain supernatants and stored at -20°C. 50 μL plasma sample and 50 μL internal standard (clozapine) working solution was vortexed with 300μL MeOH and then centrifuged. 150 μL upper organic phase was obtained and mixed with 150 μL water, followed by centrifugation. 10 μL supernatant was sampled for analysis. The tissue samples were weighted and mixed homogeneously with 10 times weight of water. Since the samples were 10-time diluted, the concentration of the tissue would be 10 times the results determined. The heart tissue was 5-time diluted. 100 μL homogenate was mixed with 50 μL internal standard (clozapine) working solution and 300μL MeOH and then centrifuged. 150 μL upper organic phase was obtained

and mixed with 150 μL water, followed by centrifugation. 10 μL supernatant was sampled for analysis. Results were shown in Fig. 2 and Tables 3, 4 and 5.

Table 3. Pharmacokinetics Study

| Parameters | Unit | Compound 1 (Sample 1) | Compound 2 (Sample 2) | Sunitinib |
|---|---|---|---|---|
| $t_{1/2}$ | h | 0.98 | 0.87 | 1.24 |
| $T_{max}$ | h | 0.08 | 0.08 | 0.08 |
| $C_{max}$ | ng/mL | 445.1 | 564.4 | 225.6 |
| $AUC_{all}$ | h*ng/mL | 477.9 | 408.8 | 364.9 |
| V | mL | 379.7 | 406.1 | 484.8 |
| Cl | mL/h | 267.5 | 324.9 | 270.6 |

Table 4. Distribution of Drug in Organs

| | AUC (h*ng/mL) | | | Cmax(ng/mL) | | |
|---|---|---|---|---|---|---|
| | Compound 1 | Compound 2 | Sunitinib | Compound 1 | Compound 2 | Sunitinib |
| Heart | 107.1 | 97.0 | 758.0 | 121.1 | 122.4 | 504.5 |
| Liver | 939.8 | 1047.1 | 2208.8 | 539.3 | 742.7 | 1091.8 |
| Spleen | 1247.8 | 664.8 | 3020.6 | 524.2 | 221.8 | 753.4 |
| Lung | 1655.9 | 1235.0 | 4393.5 | 830.8 | 763.8 | 2100.2 |
| Kidney | 495.7 | 474.2 | 3020.6 | 211.4 | 258.0 | 753.4 |
| Plasma | 477.9 | 408.8 | 364.9 | 445.1 | 564.4 | 225.6 |
| All | 4924.1 | 3926.9 | 13766.4 | 2672.0 | 2673.1 | 5428.8 |

As the pharmacokinetic parameters showed, Sunitinib had the longest half life time (1.24 h) and Compounds 1 and 2 had comparable half life time of within 1 h, indicating that the clearance of Sunitinib in Compounds 1 and 2 was quicker. The Cmax of Compounds 1 and 2 was approx. 5 times than that of the control. AUCs in all 3 compounds were comparable, indicating that the concentration-time curves of compounds 1 and 2 were steeper than that of the Sunitinib. Apparent volume of distribution (350-500 mL) suggested wider distribution in tissues.

Table 5. Parameters of Targeting

| | RE | | RTE | |
|---|---|---|---|---|
| | Compounds1 (sample 1) | Compound 2 (sample2) | Compound 1 (sample 1) | Compound 2 (sample2) |
| Heart | 0.14 | 0.13 | -0.60 | -0.55 |
| Liver | 0.43 | 0.47 | 0.19 | 0.66 |
| Spleen | 0.41 | 0.22 | 0.15 | -0.23 |
| Lung | 0.38 | 0.28 | 0.05 | -0.01 |
| Kidney | 0.16 | 0.16 | -0.54 | -0.45 |
| Plasma | 1.31 | 1.12 | 2.66 | 2.93 |

As shown in Table 5, only the plasma had a RE value greater than 1, indicating increased exposure in plasma and decreased exposure in other organs under the tested concentrations.

RTE analysis suggested that compound 1 had increased distribution proportions in liver and spleen; compound 2 had increased distribution proportions in spleen and lung, decreased proportions in heart and kidney but great enhancement in plasma.

No prodrug was detected in organs except for spleen during processing. Previous experiments showed that compounds 1 and 2 were stable in MeOH stock solution (stability test period: 7 days), while easy to degrade in the event of coexisting with biomass. The prodrugs may be converted to Sunitinib or other metabolite during processing or in vivo.

Pharmacodynamics

Since Sunitinib was orally administered to patients, we performed oral route pharmacodynamic studies in Sunitinib and compounds 1 and 2. Compounds 1 and 2 were dissolved in malate solution and adjusted to pH 7. Sunitinib was dissolved in pure water. Dosing: 20 mg/kg Sunitinib malate, 19.248 mg/kg Compound 1, and 20 mg/kg Compound 2 was gavaged nto mice, respectively (n=24 for each group). Animal experiments: 24 mice in each group were divided into 8 sub-groups. 0.25h, 0.5h, 1 h, 1 h, 1.5 h, 2 h, 4 h, 6 h, 10 h post gavage, blood was taken by eyeball removal and the mice were sacrificed by cervical dislocation. Tissues were perfused with normal saline and livers and kidneys were removed and cleaned with normal saline. The blood samples were centrifuged to obtain supernatants and stored at -20°C. Results were shown in Fig. 3 and Tables 6 and 7.

Table 6. pharmacodynamic parameters

| Parameter | Unit | Sunitinib (control) | Compound **2** | Compound **1** |
|---|---|---|---|---|
| $t_{1/2}$ | h | 4.73 | 3.89 | 2.94 |
| $T_{max}$ | h | 0.50 | 1.50 | 0.50 |
| $C_{max}$ | ng/mL | 9.58 | 27.00 | 22.70 |
| $AUC_{0\to\infty}$ | h*ng/mL | 43.1 | 61.5 | 68.4 |
| Cl | mL/h | 8101.5 | 4094.7 | 3848.9 |

Table 7. Comparison of pharmacodynamic results

| | Compound **1** | Compound **2** | Sunitinib (control) |
|---|---|---|---|
| $AUC_{po}$ | 946.2 | 951.1 | 214.2 |
| $AUC_{iv}$ | 478.9 | 408.8 | 364.9 |
| $D_{po}$ | 15.00 | 15.00 | 15.00 |
| $D_{iv}$ | 3.86 | 3.86 | 3.86 |
| F | 50.8% | 59.8% | 15.1% |

Compared to intravenous injection, intragastric gavage exhibited much more significant difference, which may be caused by the difference in gastrointestinal absorption. Compounds 1 and 2 had AUC approx. 3.5 times higher and Cmax far higher than Sunitinib. This indicated that compounds 1 and 2 improved the extent of absorption of Sunitinib. The bioavailability of compounds 1 and 2 were about 50%, much higher than that of Sunitinib (15%). Sunitinib had higher apparent volume of distribution, indicating intragastric gavage of Sunitinib were more tissue accumulative, while compounds 1 and 2 had decreased ratio of accumulation in liver.

**Example 15.** Comparison studies of pharmacodynamics and Toxicity among compounds 1, 2 and Sunitinib Cell lines and Culture

A549 human non-small cell lung cancer cell line, NCI-H460 human non-small cell lung cancer cell line, 786-0 human renal cell line, Caki-1 human renal cell line, HT-29 human colon cancer cell line, MDA-MB-231 human breast cancer cell line, and MCF-7 human breast cancer cell line was purchased from Chinese Academy of Science Shanghai Branch. PLC/PRF-5 human hepatoma cell line and Hepa1-6 mouse hepatoma cell line were obtained from Institute of Biochemistry and Cell Biology, SIBS, CAS (SIBCB). Cell lines A549, Caki-1, HT-29, MDA-MB-231, MCF-7, PLC / PRF-5 and Hepa1-6 were cultured in DMEM medium (Gibco, Life Technologies, China) and cell lines NCI-H460 and 786-0 were cultured in RPMI-1640 medium (Gibco, Life Technologies, China). 10% HI fetal bovine serum was purchased from Gibco, Life Technologies, USA and 1% penicillin/streptomycin was obtained from Hyclone.

Approx. 2,000 cells in 50 μL complete medium were plated on each well of a 96-well plate and incubated at 37°C, 5% $CO_2$ overnight. Drugs at concentrations of 0.02 to 10 μM were dissolved in 0.1%DMSO, followed by addition into each well. After incubation for 72 hours, 10μL CCK8 (Dojindo) was added into each well, followed by incubation at 37°C for another 4 hours. The OD values were obtained by TECAN Microplate Reader at 450 nm.

Processing of Compounds

For in vitro tests, compounds were directly dissolved in 10mM DMSO and stored at -20°C. For in vivo tests,

the compounds were dissolved in DMSO and neutralized by Tween 80 (DMSO: Tween = 1:1) and further neutralized by PBS (pH 7.4). The final concentrations of DMSO and Tween-80 were 5%.

Effects of MEK, ERK and STAT3 phosphorylation

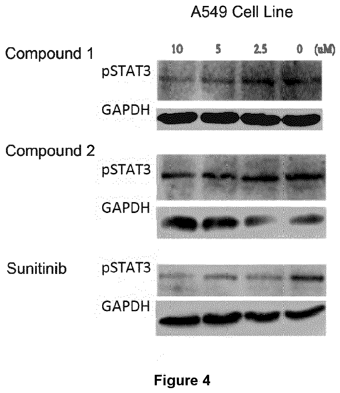

Sunitinib is an RTK inhibitor with inhibition effects against several signaling pathways, such as RAS/RAF/MAPK. We therefore analyzed some major downstream proteins including MEK, ERK and STATE. For pSTAT3, Sunitinib and prodrug 2 showed strong inhibition to A549 cell line at 10 μM (Fig. 4). These results showed that the prodrug had the same anti-tumor mechanism with Sunitinib.

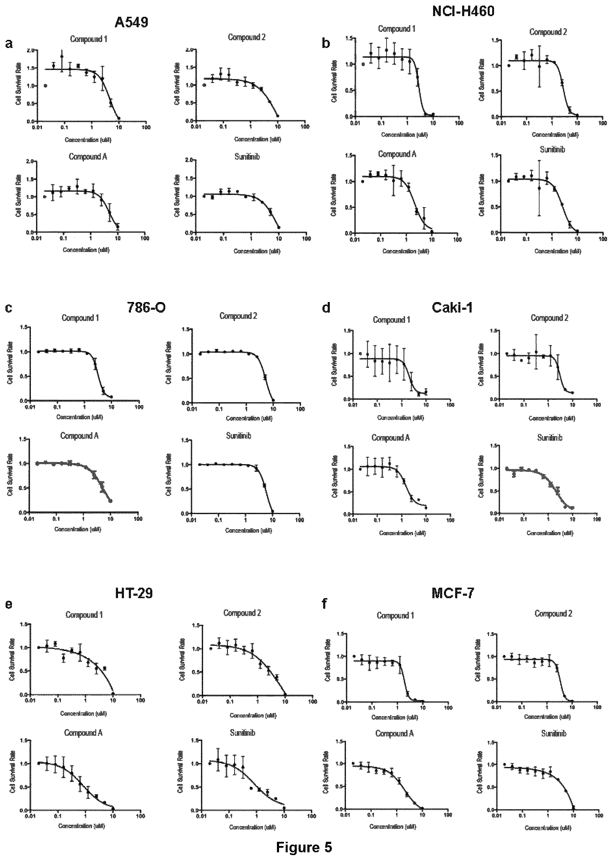

Sunitinib and the prodrugs 1 and 2 exhibited relatively strong tumor killing effects. The $IC_{50}$ values of Sunitinib and the compounds 1 and 2 were comparable among several cell lines (Fig. 5a-f). The results showed that all compounds had intense cytotoxicity.

However, in contrast to Sunitinib, cells treated by compounds 1 and 2 experienced quicker morphologic change during culture. Cancer cells incubated with compounds 1 and 2 for 4 hours changed to round and suffered from stress, while Sunitinib treated cells remained attached, elongate, and did not exhibit abnormal morphology (Fig. 9a). When incubated with compounds 1 or 2 for 8 hours, mass cell death was observed. Sunitinib treatment for 8 hours did not affect cell survival (Fig. 9b). The results indicated that compounds 1 and 2 could kill cancer cells faster than Sunitinib in an irreversible way. Compound A exhibited similar quick killing activity to compounds 1 and 2 (Fig. 9c). The in vivo target of Sunitinib is tumor vasculature endothelial cells. We therefore used human umbilical vein endothelial cells (HUVEC) to test the cytotoxicity of compounds 1 and 2. Similar to the observations for 786-0 and A549 cell lines, treatment with compounds 1 or 2 for 8 hours cause less than 50% survival of HUVEC (Fig. 9d).

Acute Toxicity Test: Maximum Tolerance Dosage (MTD) Determination

Methods: Femal BALB/c mice, 4-5 weeks old, weighted greater than 17g, were used for the test. Drugs were orally administered once a day at three dosages (28mg/kg, 70mg/kg, 175mg/kg) for 14 days. The body weights and conditions of the mice were recorded.

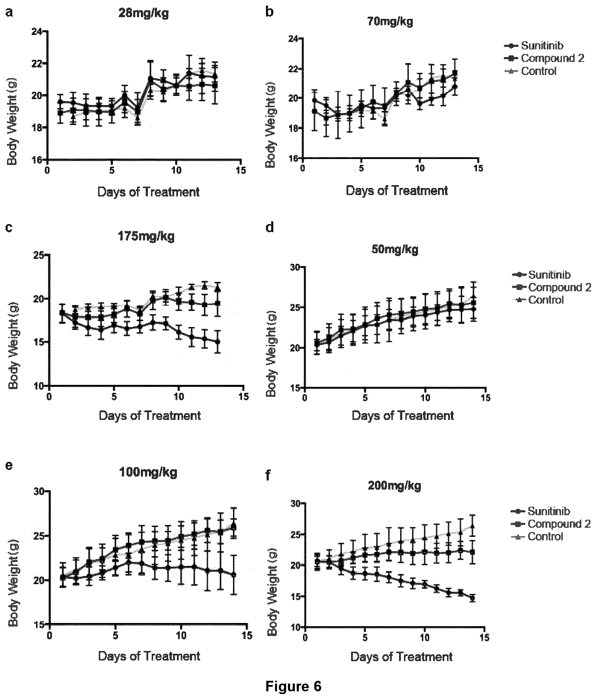

Results: Fig. 6a-c showed that Sunitinib and 3 prodrug compounds had little effects on the body weights of the animals at dosages of 28mg/kg and 70mg/kg and all mice were determined to be normal. However, when the dosage increased to 175mg/kg, severe toxicity was observed in Sunitinib group with average body weight reduce of 17.6%. In contrast, the body weights of mice in compound 2 group increased 6.0% in average. In addition, mice in Sunitinib 175 mg/kg group were extremely weak during whole test, while mice in compound 2 same dosage group performed similarly with negative control group. Therefore, the acute toxicity test indicated that mice were much more tolerate to compound 2.

The acute toxicity test was repeated by increasing the dosages: Sunitinib and 3 prodrugs were orally administered to animals at 3 different dosages (50mg/kg, 100mg/kg, 200mg/kg) once a day. It was obvious from the results (Fig. 6d-f) that at concentration of 50 mg/kg, all drugs showed little influence on body weights and all animals were determined to be normal. At 100 mg/kg, compound 2 had little influence on body weight and all animals in this group were determined to be normal. At 100 mg/kg, Sunitinib started to have influence on body weight and body weights did not increase, no decrease was detected though. However, when dosage was increased to 200 mg/kg, severe toxicity was observed in Sunitinib group with average body weight reduce of 20%. In contrast, the body weights of mice in compound 2 group increased 6.0% in average. In addition, mice in Sunitinib 200 mg/kg group were extremely weak during whole test, while mice in compound 2 same dosage group performed similarly with negative control group. Therefore, the acute toxicity test indicated that mice were much more tolerate to compound 2.

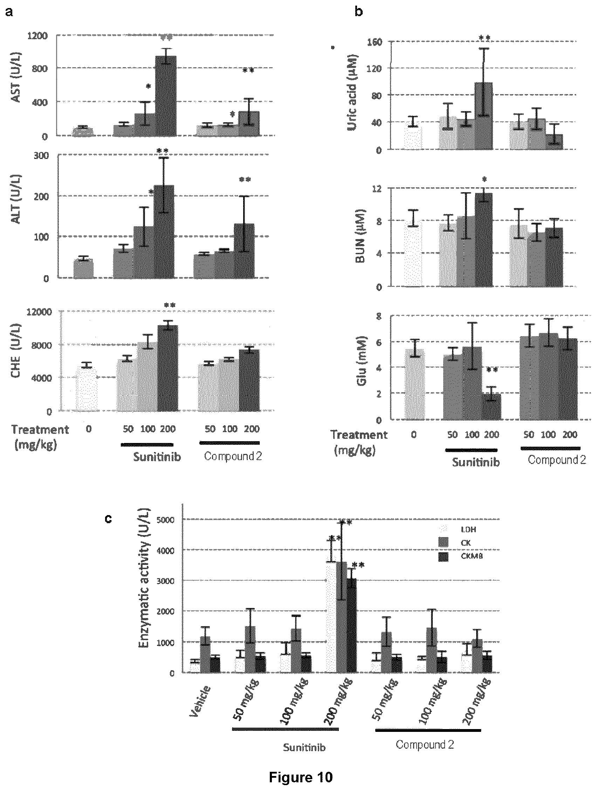

To further explore the toxicity of the compounds, blood was collected after 14-day treatment of drugs and critical biochemical index were analyzed. In 200 mg/kg Sunitinib treated mice, the levels of aspartate transaminase (AST) alanine aminotransferase (ALT) and cholinesterase (CHE) were significantly elevated (Fig. 10a), indicating possible hepatotoxicity, cardiotoxicity, nephrotoxicity, and toxicity in other tissues; the levels of urate and blood urea nitrogen was increased, while blood glucose was decreased (Fig. 10b), suggesting possible nephrotoxicity; and the levels of lactate dehydrogenase (LDH), creatine kinase (CK) and creatine kinase-MB (CKMB) were also elevated (Fig. 10c), implying possible cardiotoxicity and toxicity in other organs. In contrast, in the 200 mg/kg compound 2 treated mice, except for AST and ALT, these indices were all within normal ranges (Fig. 10c). The data further supported that compound 2 has less toxicity than Sunitinib, especially in terms of cardiotoxicity, hepatoxicity and nephrotoxicity.

Pharmacodynamic Tests on Mice Xenograft Transplantation Model

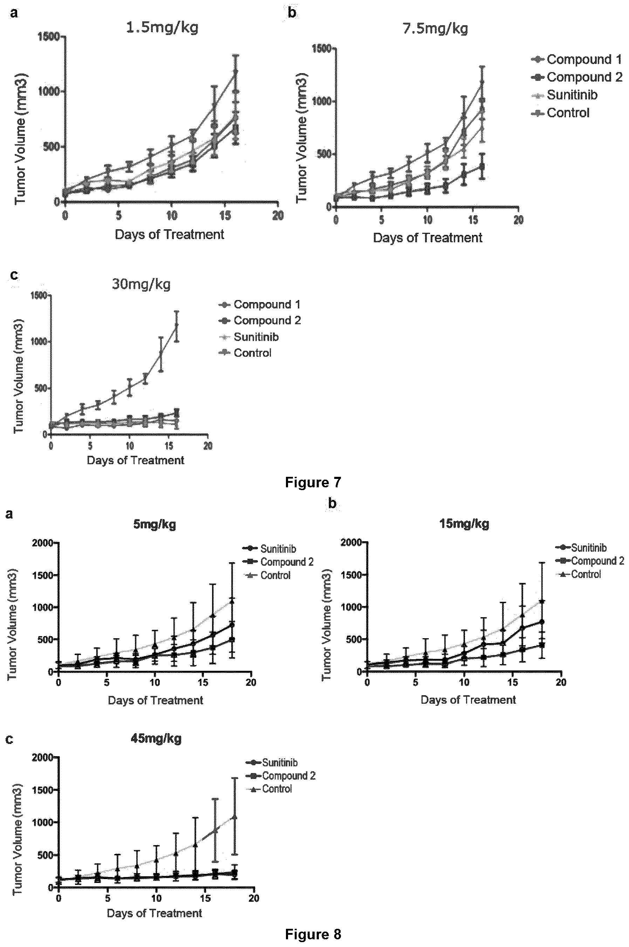

$5\times10^6$ cancer cells were subcutaneously injected to the right side of mouse. Drug was intraperitoneally injected at dosages of 1.5mg/kg, 7.5mg/kg, 30mg/kg to the mice, once a day, for 2 weeks when average tumor size reached 75 to 150 $mm^3$. The size of the tumor was measured every other day. The results (Fig. 7) showed that compound 2 had

improved efficacy than Sunitinib. Compound 1 had less efficacy.

We then performed the test through oral route. $5\times10^6$ cancer cells were subcutaneously injected to the right side of mouse. Drug was orally administered at dosages of 5mg/kg, 15mg/kg, 45mg/kg to the mice, once a day, for 2 weeks when average tumor size reached 75 to 150 $mm^3$. The size of the tumor was measured every other day. Similarly, the results (Fig. 8) showed that compound 2 had improved efficacy than Sunitinib.

**Example 16.** Tumor cell killing effects of other compounds

8-hour (short term) cell killing effects

Cell lines and Culture: Cell line A549 was cultured in DMEM medium (Gibco, Life Technologies, China). 10% HI fetal bovine serum was purchased from Gibco, Life Technologies, USA and 1% penicillin/streptomycin was obtained from Hyclone.

Approx. 2,000 cells in 50 μL complete medium were plated on each well of a 96-well plate and incubated at 37°C, 5% $CO_2$ overnight. Drugs at concentrations of 2.5 μM, 5 μM, and 10μM were dissolved in 0.1%DMSO, followed by addition into each well. After incubation for 8 hours, 10μL CCK8 (Dojindo) was added into each well, followed by incubation at 37°C for another 4 hours. The OD values were obtained by TECAN Microplate Reader at 450 nm.

Processing of Compounds: For in vitro tests, compounds were directly dissolved in 10mM DMSO and stored at -20°C.

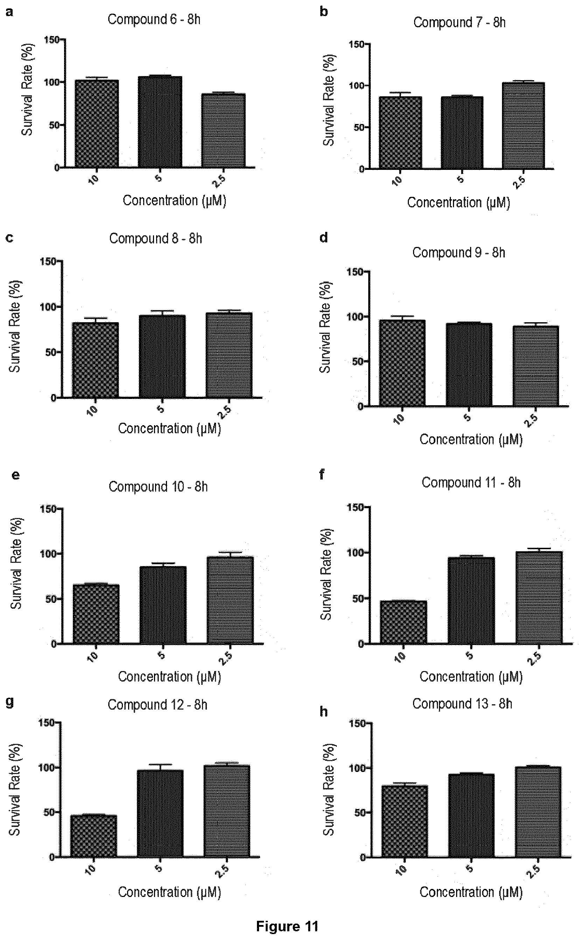

Results as shown in Fig. 11a-h indicated that short term treatment with these compounds did not show significant influence on the survival rate of the tumor cells.

Cell killing effects by 8-hour treatment followed by 24-hour recovery

Cell lines and Culture: Cell line A549 was cultured in DMEM medium (Gibco, Life Technologies, China). 10% HI fetal bovine serum was purchased from Gibco, Life Technologies, USA and 1% penicillin/streptomycin was obtained from Hyclone.

Approx. 2,000 cells in 50 μL complete medium were plated on each well of a 96-well plate and incubated at 37°C, 5% $CO_2$ overnight. Drugs at concentrations of 2.5 μM, 5 μM, and 10μM were dissolved in 0.1 %DMSO, followed by addition into each well. After incubation for 8 hours, the medium was replaced with 100 μL complete medium without drug. After incubation for 24 hours, 10μL CCK8 (Dojindo) was added into each well, followed by incubation at 37°C for another 4 hours. The OD values were obtained by TECAN Microplate Reader at 450 nm.

Processing of Compounds: For in vitro tests, compounds were directly dissolved in 10mM DMSO and stored at -20°C.

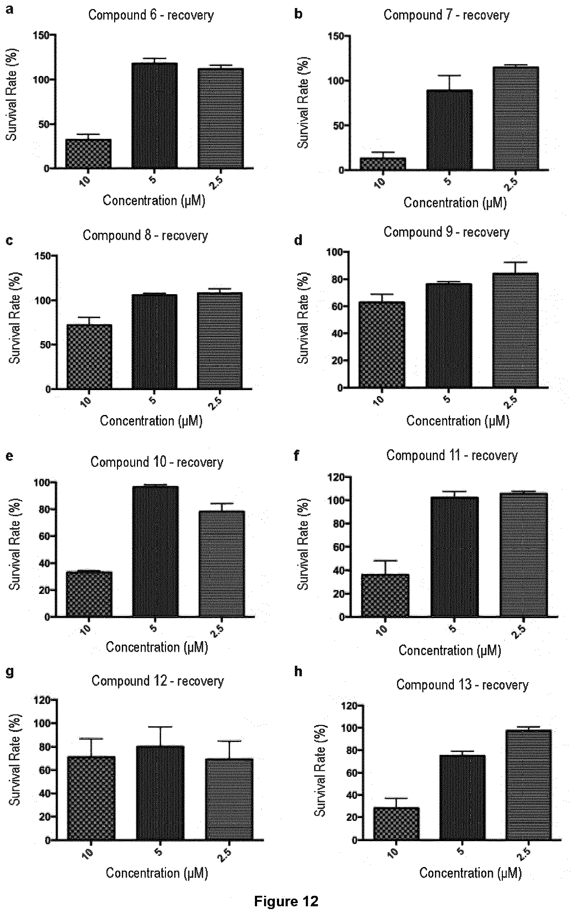

Results as shown in Fig. 12a-h indicated that short term treatment with these compounds start to affect the survival rate of the tumor cells after 24 hours and the effects were dose-dependent. The possible reasons may rely in that the compounds entered into cell at a relatively slow speed and the anti-tumor activity was delayed.

72-hour (long term) cell killing effects

Cell lines and Culture: Cell line A549 was cultured in DMEM medium (Gibco, Life Technologies, China). 10% HI fetal bovine serum was purchased from Gibco, Life Technologies, USA and 1% penicillin/streptomycin was obtained from Hyclone.

Approx. 2,000 cells in 50 μL complete medium were plated on each well of a 96-well plate and incubated at 37°C, 5% $CO_2$ overnight. Drugs at concentrations of 0.02 μM to 10μM were dissolved in 0.1% DMSO, followed by addition into each well. After incubation for 72 hours, 10μL CCK8 (Dojindo) was added into each well, followed by incubation at 37°C for another 4 hours. The OD values were obtained by TECAN Microplate Reader at 450 nm.

Processing of Compounds: For in vitro tests, compounds were directly dissolved in 10mM DMSO and stored at -20°C.

Results were shown in Table 8 and Fig. 12a-h. The results showed that those compounds showed excellent tumor inhibiting activity in a dose-dependent manner.

Table 8 - IC50 values of example compounds

| Compounds # | $IC_{50}$(μM) | Compounds # | $IC_{50}$(μM) |
|---|---|---|---|
| **6** | 8.464 | **10** | 7.456 |
| **7** | 1.788 | **11** | 2.561 |

(continued)

| Compounds # | $IC_{50}$(μM) | Compounds # | $IC_{50}$(μM) |
|---|---|---|---|
| **8** | 1.115 | **12** | 9.07 |
| **9** | 4.004 | **13** | 2.997 |

The experiment results in the foregoing demonstrated that:

(a) the Sunitinib prodrugs provided by the present invention showed better pharmacokinetics and pharmacodynamics: intravenous injection showed that compounds 1 and 2 had greater Cmax than Sunitinib, while AUC did not differ significantly; intragastric gavage administration indicated that compounds 1 and 2 had much higher Cmax and larger AUC than Sunitinib;

(b) the Sunitinib prodrugs provided herein exhibited better safety: compound 2 had higher distribution ratio in liver as compared to Sunitinib. In other organs, however, especially in heart, compound 2 had lower distribution, compared to Sunitinib. All three prodrug compounds as tested exhibited lower drug concentrations, indicating they could effectively reduce systemic toxicity, especially cardiotoxicity, suggesting better safety and sustained drug concentration; and

(c) the Sunitinib prodrugs provided herein had improved therapeutic efficacy: the Sunitinib prodrugs showed improved efficacy than Sunitinib in xenograft model in mice. The prodrugs were shown to have higher distribution in liver and lower distribution in heart, compared to Sunitinib. Moreover, the prodrugs exhibited better tumor killing effects than Sunitinib.

Overall, the Sunitinib prodrugs showed improved efficacy in cancer treatment, with lower toxicity. The prodrugs provided by the present invention are prospective in treatment of liver cancer diseases that were not clinically tested by Sunitinib. They may also serve as leading compounds of anti-tumor compounds that are efficient and low-toxic.

All patents and other references cited in the specification are indicative of the level of skill of those skilled in the art to which the invention pertains, and are incorporated by reference in their entireties, including any tables and figures, to the same extent as if each reference had been incorporated by reference in its entirety individually.

In addition, where features or aspects of the invention are described in terms of Markush groups or other grouping of alternatives, those skilled in the art will recognize that the invention is also thereby described in terms of any individual member or subgroup of members of the Markush group or other group.

## Claims

**1.** A compound having formula I:

formula (I)

or a pharmaceutically acceptable salt thereof, wherein:

$R_{12}$, $R_{13}$ are selected from the group consisting of H, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl and 5-15 membered heteroaryl;

$R_{14}$ is selected from the group consisting of R', OR', SR' and $N(R')_2$; and
R' is selected from the group consisting of H, $C_1$-$C_6$alkyl, $C_2$-$C_6$ alkenyl, $C_2$-$C_6$ alkynyl, $C_3$-$C_8$ cycloalkyl, $C_6$-$C_{10}$ aryl, 4-15 membered heterocyclyl, 5-15 membered heteroaryl, hydroxyl $C_1$-$C_6$ alkyl, carboxyl $C_1$-$C_6$ alkyl, $C_1$-$C_6$ alkyl amido and phosphate group.

**2.** The compound or a pharmaceutically acceptable salt thereof of claim 1, wherein $R_{12}$, $R_{13}$ are both H, and $R_{14}$ is selected from the group consisting of N,N-dimethylaminomethyl, t-butyl, phenyl, p-fluorophenyl, biphenyl, dimethylamino, cyclopropyl, methyl, ethyl, propyl, isopropyl, butyl, isobutyl, cyclobutyl, pentyl, isopentyl, neopentyl, cyclopentyl, 1-methylcyclobutyl, N-methylamino, N-ethylamino, N,N-methylethylamino, n-hexyl, cyclohexyl, methylthio, ethylthio, propylthio, isopropylthio, cyclopropylthio, butylthio, isobutylthio, cyclobutylthio, methoxy, ethoxy, propoxy, isopropoxy, butoxy, cyclobutoxy, pentylamino, pentylthio, pentyloxy, isopentylamino, neopentylamino, t-butylamino, cyclopentylamino, cyclopentylthio, cyclopentyloxy, cyclohexylamino, cyclohexylthio, cyclohexyloxy, p-methoxyphenyl, p-chlorophenyl and o-fluorophenyl.

**3.** The compound or a pharmaceutically acceptable salt thereof of claim 1, wherein $R_{12}$, $R_{13}$ are both H, and $R_{14}$ is t-butyl, phenyl or t-butoxy.

**4.** A compound selected from the group consisting of:

***N***-[2-(diethylamino)ethyl]-5-[(5-fluoro-2-oxo-1-(N,N-dimethylamino)methylcarboxymethyl-2,3-indolin-3-yl methylene)]-2,4-dimethyl-1***H***-pyrrolidine-3-carboxamide (Compound **A**);
***N***-[2-(diethylamino)ethyl]-5-[(5-fluoro-2-oxo-1-(*t*-butylcarboxymethyl)-2,3-indolin-3-ylmethylene)]-2,4-dimethyl-1***H***-pyrrolidine-3-carboxamide (Compound **1**);
***N***-[2-(diethylamino)ethyl]-5-[(5-fluoro-2-oxo-1-(phenylcarboxymethyl)-2,3-indolin-3-ylmethylene)]-2,4-dimethyl-1***H***-pyrrolidine-3-carboxamide (Compound **2**);
***N***-[2-(diethylamino)ethyl]-5-[(5-fluoro-2-oxo-1-(1-methylcyclobutylcarboxymethyl)-2,3-indolin-3-ylmethylene)]-2,4-dimethyl-1***H***-pyrrolidine-3-carboxamide (Compound **3**);
***N***-[2-(diethylamino)ethyl]-5-[(5-fluoro-2-oxo-1-(N-methylaminocarboxymethyl)-2,3-indolin-3-ylmethylene)]-2,4-dimethyl-1***H***-pyrrolidine-3-carboxamide (Compound **4**);
***N***-[2-(diethylamino)ethyl]-5-[(5-fluoro-2-oxo-1-(N-ethylaminocarboxymethyl)-2,3-indolin-3-ylmethylene)]-2,4-dimethyl-1***H***-pyrrolidine-3-carboxamide (Compound **5**);
***N***-[2-(diethylamino)ethyl]-5-[(5-fluoro-2-oxo-1-(N,N-dimethylaminocarboxymethyl)-2,3-indolin-3-ylmethylene)]-2,4-dimethyl-1***H***-pyrrolidine-3-carboxamide (Compound **6**);
***N***-[2-(diethylamino)ethyl]-5-[(5-fluoro-2-oxo-1-((t-butoxycarboxymethyl)-2,3-indolin-3-ylmethylene)]-2,4-dimethyl-1***H***-pyrrolidine-3-carboxamide (Compound **7**);
***N***-[2-(diethylamino)ethyl]-5-[(5-fluoro-2-oxo-1-(ethoxycarboxymethyl)-2,3-indolin-3-ylmethylene)]-2,4-dimethyl-1***H***-pyrrolidine-3-carboxamide (Compound **8**);
***N***-[2-(diethylamino)ethyl]-5-[(5-fluoro-2-oxo-1-(cyclohexylcarboxymethyl)-2,3-indolin-3-ylmethylene)]-2,4-dimethyl-1***H***-pyrrolidine-3-carboxamide (Compound **9**);
***N***-[2-(diethylamino)ethyl]-5-[(5-fluoro-2-oxo-1-(methylcarboxymethyl)-2,3-indolin-3-ylmethylene)]-2,4-dimethyl-1***H***-pyrrolidine-3-carboxamide (Compound **10**);
***N***-[2-(diethylamino)ethyl]-5-[(5-fluoro-2-oxo-1-(cyclopropylcarboxymethyl)-2,3-indolin-3-ylmethylene)]-2,4-dimethyl-1***H***-pyrrolidine-3-carboxamide (Compound **11**);
***N***-[2-(diethylamino)ethyl]-5-[(5-fluoro-2-oxo-1-(isopropylcarboxymethyl)-2,3-indolin-3-ylmethylene)]-2,4-dimethyl-1***H***-pyrrolidine-3-carboxamide (Compound **12**); and
***N***-[2-(diethylamino)ethyl]-5-[(5-fluoro-2-oxo-1-(p-methoxyphenylcarboxymethyl)-2,3-indolin-3-ylmethylene)]-2,4-dimethyl-1***H***-pyrrolidine-3-carboxamide (Compound **13**),
or a pharmaceutically acceptable salt thereof.

**5.** A pharmaceutical composition, comprising an effective amount of a compound or a pharmaceutically acceptable salt thereof of any of claims 1-4, and a pharmaceutically acceptable carrier.

**6.** Use of a compound or a pharmaceutically acceptable salt thereof of any of claims 1-4 in preparation of a drug for treating a cancer.

**7.** The use of claim 6, wherein the cancer is metastatic renal cell carcinoma, gastrointestinal stromal tumor or liver cancer.

Concentration-time curves of Sunitinib in mice plasma after caudal vein administration of two compounds
Sunitinib Concentration/(ng/mL)
700
600
500
400
300
200
100
0
0
2
4
6
8
10
Time (h)
Compound A
Sunitinib

Figure 1

a
Concentration-Time Curve
Concentration (ng/ml)
Time (h)
Compound 1
Compound 2
Sunitinib
b
Concentration-Time Curve
Concentration (ng/ml)
Time (h)
Compound 1
Compound 2
Sunitinib

Figure 3

a
Concentration-Time Curve (Heart)
Compound 1
Compound 2
Sunitinib
Concentration (ng/ml)
Time (h)
b
Concentration-Time Curve (Liver)
Compound 1
Compound 2
Sunitinib
Concentration (ng/ml)
Time (h)
c
Concentration-Time Curve (Spleen)
Compound 1
Compound 2
Sunitinib
Concentration (ng/ml)
Time (h)
d
Concentration-Time Curve (Lung)
Compound 1
Compound 2
Sunitinib
Concentration (ng/ml)
Time (h)
e
Concentration-Time Curve (Kidney)
Compound 1
Compound 2
Sunitinib
Concentration (ng/ml)
Time (h)
f
Concentration-Time Curve (Plasma)
Compound 1
Compound 2
Sunitinib
Concentration (ng/ml)
Time (h)

Figure 2

A549 Cell Line

Compound 1 10 5 2.5 0 (uM)

pSTAT3

GAPDH

Compound 2

pSTAT3

GAPDH

Sunitinib pSTAT3

GAPDH

Figure 4

A549
NCI-H460
786-O
Caki-1
HT-29
MCF-7
a
b
c
d
e
f
Compound 1
Compound 2
Compound A
Sunitinib
Cell Survival Rate
Concentration (uM)

Figure 5

a
28mg/kg
Body Weight (g)
Days of Treatment
b
70mg/kg
Body Weight (g)
Days of Treatment
Sunitinib
Compound 2
Control
c
175mg/kg
Body Weight (g)
Days of Treatment
d
50mg/kg
Body Weight (g)
Days of Treatment
Sunitinib
Compound 2
Control
e
100mg/kg
Body Weight (g)
Days of Treatment
f
200mg/kg
Body Weight (g)
Days of Treatment
Sunitinib
Compound 2
Control

Figure 6

a
1.5mg/kg
Tumor Volume (mm3)
Days of Treatment
b
7.5mg/kg
Compound 1
Compound 2
Sunitinib
Control
c
30mg/kg
Figure 7
a
5mg/kg
Sunitinib
Compound 2
Control
b
15mg/kg
c
45mg/kg

Figure 8

a
Sunitinib
Compound 1
Compound 2
b
Compound 1
Compound 2
Sunitinib
Survival Rate (%)
786-O cells
A549 cells
10 µM
5µM
2.5 µM
c
Survival Rate (%)
Compound 1
Compound 2
Compound A
Sunitinib
Concentration (µM)
d
HUVEC cells
Compound 1
Compound 2
Sunitinib
Survival Rate (%)
10 µM
5µM
2.5 µM

Figure 9

a
AST (U/L)
ALT (U/L)
CHE (U/L)
Treatment (mg/kg)
0
50 100 200
Sunitinib
Compound 2
b
Uric acid (μM)
BUN (μM)
Glu (mM)
c
Enzymatic activity (U/L)
LDH
CK
CKMB
Vehicle
50 mg/kg
100 mg/kg
200 mg/kg

**Figure 10**

a
Compound 6 - 8h
Survival Rate (%)
Concentration (μM)
b
Compound 7 - 8h
Survival Rate (%)
Concentration (μM)
c
Compound 8 - 8h
Survival Rate (%)
Concentration (μM)
d
Compound 9 - 8h
Survival Rate (%)
Concentration (μM)
e
Compound 10 - 8h
Survival Rate (%)
Concentration (μM)
f
Compound 11 - 8h
Survival Rate (%)
Concentration (μM)
g
Compound 12 - 8h
Survival Rate (%)
Concentration (μM)
h
Compound 13 - 8h
Survival Rate (%)
Concentration (μM)

Figure 11

a
Compound 6 - recovery
Survival Rate (%)
150
100
50
0
10
5
2.5
Concentration (μM)
b
Compound 7 - recovery
Survival Rate (%)
150
100
50
0
10
5
2.5
Concentration (μM)
c
Compound 8 - recovery
Survival Rate (%)
150
100
50
0
10
5
2.5
Concentration (μM)
d
Compound 9 - recovery
Survival Rate (%)
100
80
60
40
20
0
10
5
2.5
Concentration (μM)
e
Compound 10 - recovery
Survival Rate (%)
100
80
60
40
20
0
10
5
2.5
Concentration (μM)
f
Compound 11 - recovery
Survival Rate (%)
120
100
80
60
40
20
0
10
5
2.5
Concentration (μM)
g
Compound 12 - recovery
Survival Rate (%)
120
100
80
60
40
20
0
10
5
2.5
Concentration (μM)
h
Compound 13 - recovery
Survival Rate (%)
120
100
80
60
40
20
0
10
5
2.5
Concentration (μM)

**Figure 12**

a
Compound 6
Survival Rate (%)
Concentration (μM)
b
Compound 7
Survival Rate (%)
Concentration (μM)
c
Compound 8
Survival Rate (%)
Concentration (μM)
d
Compound 9
Survival Rate (%)
Concentration (μM)
e
Compound 10
Survival Rate (%)
Concentration (μM)
f
Compound 11
Survival Rate (%)
Concentration (μM)
g
Compound 12
Survival Rate (%)
Concentration (μM)
h
Compound 13
Survival Rate (%)
Concentration (μM)

Figure 13

# INTERNATIONAL SEARCH REPORT

International application No. PCT/CN2016/071951

**A. CLASSIFICATION OF SUBJECT MATTER**

C07D 403/06 (2006.01) i; A61K 31/404 (2006.01) i; A61P 35/00 (2006.01) i; A61P 1/16 (2006.01) i

According to International Patent Classification (IPC) or to both national classification and IPC

B. FIELDS SEARCHED

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI; EPODOC; CNPAT; CNKI; CA; STN(REG, CAPLUS): C07D 403/06, A61K 31, sunitinib, prodrug, kinase, inhibitor, Preparation, Anticancer, cancer

C. DOCUMENTS CONSIDERED TO BE RELEVANT

| Category* | Citation of document, with indication, where appropriate, of the relevant passages | Relevant to claim No. |
|---|---|---|
| X | Buchy Eric et al., Synthesis and Cytotoxic Activity of Self-Assembling Squalene Conjugates of 3-[(Pyrrol-2-yl) methylidene]-2, 3-dihydro-1H-indol-2-one Anticancer Agents, European Journal of Organic Chemistry, No.1, page 202-212, 21 November 2014 (21.11.2014); see page 202, compounds 4 and page 206, the right column | 1-7 |
| A | CN 103848985 A (YANG, Zijian) 11 June 2014 (11.06.2014) see the whole document | 1-7 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 12 April 2016 | 28 April 2016 |

Name and mailing address of the ISA
State Intellectual Property Office of the P. R. China
No. 6, Xitucheng Road, Jimenqiao
Haidian District, Beijing 100088, China
Facsimile No. (86-10) 62019451

Authorized officer
LIU, Yanming
Telephone No. (86-10) 62089194

Form PCT/ISA /210 (second sheet) (July 2009)

**INTERNATIONAL SEARCH REPORT**
Information on patent family members

International application No.
PCT/CN2016/071951

| Patent Documents referred in the Report | Publication Date | Patent Family | Publication Date |
|---|---|---|---|
| CN 103848985 A | 11 June 2014 | None | |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201510045099